# EUROPEAN PATENT APPLICATION

(11) **EP 1 995 328 A1**
(43) Date of publication of application: **26.11.2008**
(21) Application number: 07108612.8
(22) Date of filing: 22.05.2007
(51) Int. Cl.: C12Q 1/68

(54) **Method of detecting probe-target binding**

(71) Applicant: Koninklijke Philips Electronics N.V., 5621 BA Eindhoven (NL)
(72) Inventor: The designation of the inventor has not yet been filed
(74) Representative: Van Velzen, Maaike Mathilde

(57) **Abstract**

This invention describes a method for the separation of unbound labelled probe and probe/target complex in solution, by manipulation of probe and/or probe/target complex. Using these methods, optical detection of the probe/target complex can be performed within the reaction chamber and allows recycling of the unbound probe. The methods are applicable to proteins, nucleic acids and other biological molecules. The method is particularly suited for real-time quantitative PCR.

## Description

### FIELD OF THE INVENTION

The invention relates to methods of separating unbound labelled probes from probe/target complex so as to allow detection of probe/target binding.

### BACKGROUND OF THE INVENTION

The detection of biological molecules with binding assays is based on the binding of a detectable probe to a target (and generation of a probe/target complex). Generally both the unbound probe and the probe/target complex generate the same signal, such that these need to be separated so as to allow detection of binding. This is the case for example when probes with a fluorescent label are used. Physical separation of unbound probe molecules can be achieved by e.g. gel electrophoresis, magnetic bead separation or spin filters. However, all these procedures are time consuming and in addition only a part of the original sample is recovered, typically about 60-80%.

Alternatively, the probe or label may be designed to only give a signal, or give a different signal in the bound state. For fluorescent detection of DNA this can be accomplished by using intercalating dyes, which change their fluorescence spectrum upon binding double-stranded (ds) DNA. However, intercalating dyes can not be used for simultaneous detection of multiple probes (multiplexing), since they bind unspecific to all DNA.

Alternatively, a second label can be introduced that quenches the fluorescence of the first dye when both are in close proximity. For detection of DNA, the probe can be designed in such a way that the labels are separated upon binding of the probe to the target, such that fluorescence intensity increases. Widely applied examples for these probes are molecular beacons and Taqman probes [Tyagi et al. (1998) Nature Biotechnology 16, 49; Livak et al. (1995) PCR Methods Appl. 4, 357]. Probes containing a second dye or quencher are suited for multiplex detection, but the introduction of the second label approximately doubles the cost of the probe. Dye-quencher probes are also more difficult to design than probes containing only one label.

A third approach typically applied in nucleic acid binding uses heterogeneous assays, wherein the biological molecules in a sample are immobilized on a solid surface (e.g. cDNA microarrays). In these methods, unhybridized probes can simply be washed away. The main disadvantage of this approach is the significantly longer time needed for hybridisation due to slow diffusion processes as compared to homogeneous assays (i.e. in solution).

Methods that allow separation of unbound probe and probe/target complex in solution would greatly simplify the design of binding assays and the detection of target molecules.

US20060166249 describes a method wherein nanoparticles are used that have the property to bind preferentially to single strand (ss) nucleic acid probes. These nanoparticles are added after the formation of a soluble probe-target nucleic acid complex. The nanoparticle/ss probe aggregates are subsequently removed or precipitated, whereafter detection of the soluble probe-target complex takes place. This method is however limited to nucleic acids and is not applicable in assays wherein excess probe is re-used for interaction with the target nucleic acid, such as in real-time PCR methods.

There remains a need for binding assays wherein the presence of unbound probe in a reaction chamber does not interfere with the probe/target complex during optical detection and wherein probe, target and probe/target complex remain in solution.

### OBJECT AND SUMMARY OF THE INVENTION

Particular and preferred aspects of the invention are set out in the accompanying independent and dependent claims. Features from the dependent claims may be combined with features of the independent claims and with features of other dependent claims as appropriate and not merely as explicitly set out in the claims.

The methods and tools of present invention involve the removal of soluble unbound probe molecules from a soluble probe/target complex using a single separation step. In this way the separation process can be accelerated from hours to minutes.

The methods of the present invention take place in solution, which allows probe/target binding (e.g. nucleic acid hybridisation) to take place within seconds as compared to hours needed for hybridisation to surface bound targets/probes (e.g. microarray techniques).

The methods of the present invention are carried out within one reaction chamber, optionally comprising the original sample, such that sample loss is avoided.

The methods of the present invention allow multiple probes labelled with different optically active dyes (e.g. for fluorescence or Raman spectroscopy) to be bound and separated from the unbound probes at the same time, which allows performing multiplex assays.

The methods of the present invention allow the use of probes with only one chromophoric label, which are considerably cheaper and easier to design than molecular beacons or Taqman probes.

A first aspect of the invention relates to methods for detecting the presence of a target in a sample using a detection probe, which essentially comprise manipulating the bound and/or the unbound detection probe such that they are separated, so that either the bound or unbound detection probe can be detected. According to particular embodiments, the methods comprise the steps of:
a) contacting the sample comprising the target with a optically labelled detection probe in a sample container comprising a detection zone,
b) allowing the probe to bind with the target so as to form a probe/target complex,
c1) manipulating the probe/target complex or unbound probe to the detection zone, and/or
c2) manipulating unbound probe or probe/target complex, respectively away from the detection zone, and
d) optically detecting the probe/target complex or the unbound probe as a measure of the amount of target in the sample. Advantageously, the methods of the present invention allow detection under conditions whereby the probe/target complex and the unbound probe remain in solution within the sample container.

In particular embodiments of the methods of the invention, the manipulation steps c1 and/or c2 are performed by applying an electric field on the reaction chamber such that molecules (i.e. unbound probe or probe/target complex) in the reaction chamber are moved towards and/or away from the detection zone.

In further particular embodiments of the methods of the invention the target is provided with a manipulatable label prior to step (a). In these embodiments, the manipulation steps c1 and/or C2 are performed by applying a force to the reaction chamber such that molecules comprising the manipulatable label are moved towards/away from the detection zone.

In further particular embodiments, the methods of the invention comprise the step of contacting the target with a manipulation probe comprising a manipulatable label. The detection probe and the manipulation probe each bind specifically to a different region of the target, allowing simultaneous binding of the target to both the manipulation probe and the detection probe.

In particular embodiments of the methods of the invention, the manipulatable label is a magnetic particle.

In another particular embodiment of the method, the manipulatable label is a glass or polystyrene bead. These glass or polystyrene beads can be manipulated e.g. with a light beam.

In particular embodiments of the methods of the invention, the target and the optically labelled probe (or detection probe)(and, where applicable the manipulation probe) are nucleic acids.

In further particular embodiments, the target nucleic acid is amplified by PCR prior to step (a). Most particularly, a PCR primer pair is used of which one primer comprises a chromophoric label and the other primer comprises a manipulatable label. Herein the probe/target complex is comprised within the amplified target.

In alternative particular embodiments of the methods of the invention, the target is a peptide or protein.

In particular embodiments, the detection (and manipulation) probe is an antibody or antigen binding part thereof, a peptide with protein binding properties or an organic molecule with protein binding properties. In further particular embodiments the target is provided with a manipulatable label by binding with a probe comprising glass or polystyrene beads or by binding with a probe comprising magnetic beads.

Another aspect of the present invention relates to a primer pair for performing a PCR reaction wherein one primer of the pair comprises a chromophoric label and wherein the second primer comprises a manipulatable label.

In particular embodiments of this aspect of the invention, the manipulatable label is a magnetic particle.

Another aspect of the invention relates to devices (1) for qualitatively and/or quantitatively detecting a target in a sample, which comprise:
- an optionally removable reaction chamber (5), suitable for containing a fluid sample, comprising a separate detection zone (52) and a non-detection zone or control detection zone (51),
- manipulation means (2) spanning the detection zone (52) and the non-detection or control detection zone (51) of the reaction chamber, and
- means for optical detection (3) in said detection zone (52).

In a particular embodiments, the manipulation means (2) is a means for applying an electric or magnetic field between the detection zone (52) and the non-detection zone (51). In other particular embodiments the manipulation means (2) is a means for applying a light beam to move the manipulatable label between the detection zone (52) and the non-detection zone (51).

In further particular embodiments of the devices of the invention, the non-detection zone and the detection zone are comprised within separate chambers, which are fluidly connected.

In particular embodiments, the devices of the invention further include an element for cooling and heating the reaction chamber between 4 and 100 °C.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference Figures quoted below refer to the attached drawings.

### BRIEF DESCRIPTION OF THE FIGURES

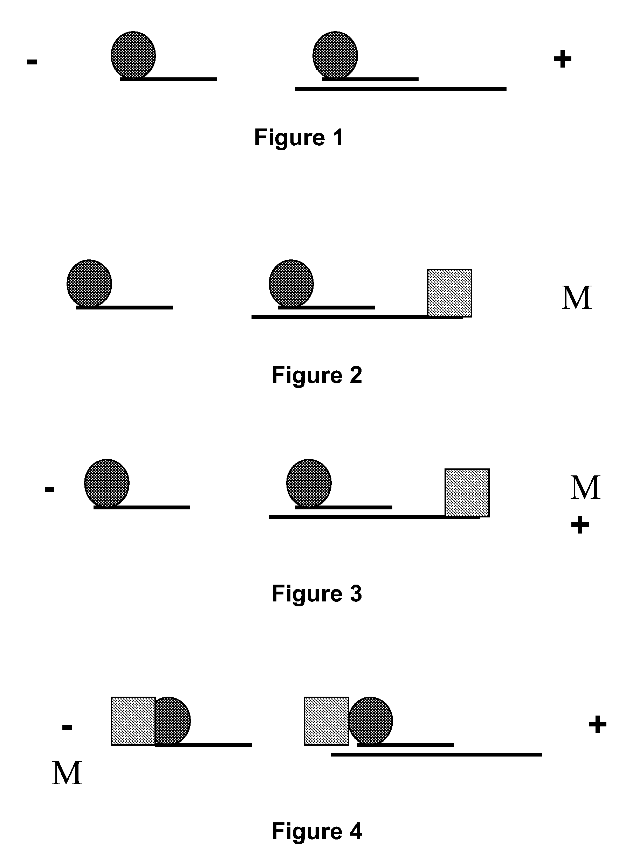

Figures 1-6 show schematic overviews of particular embodiments of the methods of the present invention. The shorter line represents a probe carrying a chromophoric label (circle). The longer line represents a target molecule. The grey square and diamond are manipulatable labels. M is an external applied force which attracts the manipulatable label. - + indicates an electric field.
In Figure 1, no manipulatable label is attached and unbound probe and probe/target complex are separated based on their intrinsic charge in an electric field.
In Figure 2, a manipulatable label is attached to the target molecule. The probe/target complex is manipulated by the applied force M.
In Figure 3, a manipulatable label is attached to the target molecule. The probe/target complex is manipulated by the applied force M. The unbound probe is manipulated in an electric field.
In Figure 4, a manipulatable label is attached to the probe. The probe/target complex and the probe are manipulated by the applied force M. A electric force larger than M is applied to move the probe/target complex in a different direction than the unbound probe.
In Figure 5, two different manipulatable labels are attached to the probe and the target molecule. The probe/target complex and the probe are manipulated by the applied forces M1 and M2. The force M2 is larger than M1 to move the probe/target complex in a different direction than the unbound probe.
Figure 6 shows a particular embodiment wherein two PCR primers are used, one with a chromophoric label and the other with a manipulatable label (A). PCR amplification (B) results in an amplified product which incorporated both labels. Upon application of an external field and an electric field, unbound probe and probe/target complex are separated.
Figure 7 shows a schematic representation of a device (1) for separating unbound chromophoric labelled from a probe/target complex comprising means for manipulation of biological molecules (2) and means for chromophoric detection (3), wherein said device is adapted to receive a reaction chamber (5) in a reaction chamber holder (4) wherein a biological molecule is present in solution.
Figure 8 shows a schematic overview of a particular embodiment of the methods of the present invention. Panel A shows the binding of chromophoric probe to a target nucleic acid in the hybridisation chamber (51) of the reaction chamber (5). Panel B shows the manipulation of labelled probe/target complex to the detection compartment (52), followed by optical detection (panel C) of the chromophoric group on the probe/target complex.

In the different Figures, the same reference signs refer to the same or analogous elements.

### DESCRIPTION OF EMBODIMENTS

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention. These definitions should not be construed to have a scope less than understood by a person of ordinary skill in the art.

The term **"target molecule"** in the context of the present invention relates to a biological molecule which presence and concentration in a sample is to be determined. It includes but is not limited to proteins, peptides (50 amino acids or less), nucleic acids (e.g. DNA and RNA), carbohydrates, lipids.

The term **"probe"** in the context of the present invention relates to a molecule, which specifically binds to a target molecule.

The term **"optically labelled probe"** in the context of the present invention relates to a probe which comprises an chromophoric label.

The term **"probe/target complex"** refers to a complex resulting from the specific binding of a probe to a target. This includes the binding of a nucleic acid probe, which has been incorporated into a DNA strand by PCR, as a complementary strand to the target.

The term **"reaction chamber"** as used herein refers to a chamber suitable for comprising a solution wherein a probe and target in solution can be contacted.

The term **"detection zone"** when referring to a reaction chamber herein refers to a region within the reaction chamber wherein the detection of the presence of a chromophoric label takes place. The term "non-detection zone" when referring to a reaction chamber refers to a region within the reaction chamber which is physically separated from the detection zone. The detection zone and non-detection zone of a reaction chamber are fluidly connecting zones.

The term **"manipulatable label"** in the context of the present invention relates to a molecule or particle, which can be manipulated by application of an external force or field (electric or magnetic force, light beam tweezers, centrifugal force, etc.).

The term **"manipulation probe"** as used herein refers to a target-specific probe or primer which comprises a manipulatable label.

The term **"detection probe"** as used herein refers to a target-specific probe or primer which comprises a chromophoric label.

The present invention relates to techniques for qualitatively and/or quantitatively detecting the presence of a target molecule in a sample using chromophoric labelled probes, wherein unbound probe and probe/target complex remain in solution within the same buffer during the analysis, at least up to the point of optical detection.

The present invention relates to methods wherein a probe/target complex and unbound probe are physically separated in solution within a reaction chamber. This is performed by providing a reaction chamber with a detection zone and a non-detection zone and manipulating the probe/target complex and/or unbound probe to different zones within the reaction chamber. In one embodiment, the probe/target complex is manipulated towards the detection zone. Alternatively, the unbound probe is manipulated to a non-detection zone. In yet another alternative, both probe/target complex and unbound probe are manipulated, respectively towards and away from the detection zone in the reaction chamber. In further alternative embodiments the detection of the target is based on the remaining unbound probe after binding of the probe with the target. In these methods, based on detection of the unbound probe, the unbound probe is manipulated towards the detection zone and/or the probe/target complex is manipulated away from the detection zone in the reaction chamber.

The present invention solves the problem of differentiating between probes with a chromophoric label, which are bound to a target (resulting in a probe/target complex) and the same probes which is not bound to the target molecule (unbound probe) in solution. Optical detection generally can not discriminate between a bound and an unbound chromophoric labelled probe and thus cannot differentiate the background contribution from the signal. The methods of the present invention make it possible to discriminate between bound and unbound labelled probe, without immobilisation or washing steps. The entire analysis method is performed within the same reaction chamber, optionally in the same sample solution, wherein probe, target and probe/target complex remain in solution.

The present invention is applicable to a wide variety of samples and target molecules present in solution therein. Target molecules which can be detected with the present methods comprise nucleic acids, but also proteins, carbohydrates, (phospho)lipids, and other biological molecules, such as metabolites. The solubilisation of a target within a sample can be improved by methods known by the skilled person such as adjusting pH or salt concentrations or adding compounds which enhance solubility, such as detergents, chaotropic agents (e.g. urea) or organic compounds (e.g. alcohol or ethylene glycol). Alternatively, the solubility of a target molecule can be enhanced by chemical modification of functional groups of a target molecule with hydrophilic groups.

In the methods of the present invention, unbound probe and probe/target complex are physically separated. This separation is performed either by exploiting the intrinsic properties of the probe and/or probe/target complex or by providing the target and/or the probe by a manipulatable label which allows physical manipulation.

Accordingly, certain embodiments of the methods and tools of the present invention make use of manipulatable labels, which, depending on the embodiment, are provided on the probes and/or the target molecules used in the methods of the invention. Manipulatable labels are labels which can be subjected to an external force or field such that they are moved within a solution, more particularly within a solution in a reaction chamber, from one zone of the reaction chamber to another. Examples of such manipulatable labels include, but are not limited to charged groups (e.g. polylysine, polyarginine, spermine, spermidine) or particles (e.g. polystyrene particles coated with charged groups), which can be manipulated by applying an electric field, magnetic particles which can be manipulated by applying a magnetic field, glass or polystyrene beads which can be manipulated with socalled optical tweezers or particles (such as polystyrene beads of a predefined size) which can be manipulated by applying a suitable centrifugational force.

According to particular embodiments, the methods of the invention involve the use of a probe to which a manipulatable label has been attached. Suitable methods for the attachment of the manipulatable labels according to the present invention to a target molecule or a probe according to the present invention, will depend on the nature of the label and the probe or target. Methods for attaching charged molecules or particles, magnetic particles, and/or glass beads or other suitable labels to DNA, proteins and other biological molecules are described in the art in e.g. Mehta et al. (1999) Science. 283, 1689-1995, W02006131892, WO2005010527 or US5512439.

In a particular embodiment of the methods of the present invention, the target is provided with a manipulatable probe. Where the target is a nucleic acid molecule this is typically ensured by amplification of the target and incorporation of the manipulatable label by the use of a primer to which a manipulatable label has been attached. The generation of primers comprising a manipulatable label can be performed using one of the methods referred to above. Alternatively, the manipulatable probe is attached to the target molecule within the sample directly using one of the methods described above.

The methods of the present invention are based on the detection of chromophoric labels, i.e. groups which emit light in the UV part or IR part, but particularly in the visible region of the spectrum i.e. between 400 and 750 nm. Suitable probes include fluorescent labelled probes and probes labelled for Raman spectroscopy. Fluorescent labels include but are not limited to fluorescein isothiocyanates (FITC), carboxyfluoresceins such as tetramethylrhodamine (TMR), carboxy tetramethyl-rhodamine (TAMRA), carboxy-X-rhodamine (ROX), sulforhodamine 101 (Texas red™), Atto dyes (Sigma Aldrich), Fluorescent Red and Fluorescent Orange, phycoerythrin, phycocyanin, and Crypto-Fluor™ dyes, etc.

Where the detection is based on SE(R)RS, the label is a material which is SE(R)RS-active, i.e. which is capable of generating a SERS or SERRS spectrum when appropriately illuminated, also referred to herein as a SE(R)RS-active label or dye. Non-limiting examples of SE(R)RS-active labels include fluorescein dyes, such as 5- (and 6-) carboxy-4',5'-dichloro-2',7'-dimethoxy fluorescein, 5-carboxy-2',4',5',7'-tetrachlorofluorescein and 5-carboxyfluorescein, rhodamine dyes such as 5- (and 6-) carboxy rhodamine, 6-carboxytetramethyl rhodamine and 6-carboxyrhodamine X, phthalocyanines such as methyl, nitrosyl, sulphonyl and amino phthalocyanines, azo dyes, azomethines, cyanines and xanthines such as the methyl, nitro, sulphano and amino derivatives, and succinylfluoresceins. Each of these may be substituted in any conventional manner, giving rise to a large number of useful labels.

These labelled nucleic acid probes are commercially available from companies such as Invitrogen, Roche Molecular Biochemicals, Promega Corp, Ambion Inc or GE Healthcare or can be made as described in e.g. US4,962,037, US5,405,747, US6,136,543 and US6,210,896.

Another suitable type of label for chromophoric detection are quantum dots (commercially available from e.g. Evident Technologies). These are crystalline semiconductor clusters typically derived in the II/VI and III/V material systems. The most commonly used semiconductors are CdSe, CdS, HgTe, CdTe, InP and InAs. The conjugation of quantum dots to DNA and RNA is described for example in Xu et al. (2003) Nucleic Acids Res. 31, e43. and Liang et al. (2005) Nucleic Acids Res. 33, e17.

In the methods of the present invention, the chromophoric labels are typically attached to the probe, so as to generate, upon contacting of the target with the probe a labelled probe/target complex.

According to a particular embodiment, the methods of the present invention are specifically designed for the detection of nucleic acids. Nucleic acids envisaged to be detected using the methods of the present invention include both single stranded and double stranded nucleic acids, and can be DNA, RNA, or PNA or can be DNA/PNA, DNA/RNA or PNA/RNA hybrids. However as detailed below, the same principles of manipulating probes, target molecules and probe/target complexes are applicable to methods involving the detection of proteins or other biological molecules.

As previously stated, the detection methods and tools of the present invention are based on the binding of a labelled probe to a target and the manipulation of the resulting probe/target complex and/or remaining unbound probe, such that these are physically separated.

As detailed above, in particular embodiments of the tools and methods of the invention, the manipulation ensuring physical separation of probe/target complex and labelled probe is performed using the intrinsic properties of bound versus unbound probes. Nucleic acids have an electric charge due to the presence of phosphate molecules. Accordingly, nucleic acids can be moved within a gel or a solution by applying an appropriate electric field. It is this property that is also used in DNA electrophoresis. As all nucleic acids have a negative charge, they will all migrate in the same direction (i.e. the direction of the positive electrode) when a direct electrical current is applied. According to particular embodiments of the methods of the present invention, an electric field is applied to the solution in the reaction chamber such that nucleic acids are manipulated from one zone of the reaction chamber to another.

In the methods according to this embodiment of the invention, an electric field is applied to the reaction chamber comprising unbound labelled nucleic acid probes, nucleic acid target and probe/target complex. Upon application of the electric field, unbound nucleic acid probes, nucleic acid target and probe/target complex present in solution all migrate towards the positive electrode. However, as a result of the charge differences between singlestranded and double-stranded DNA and between DNA molecules with different lengths, these molecules move towards the positive electrode at a different speed. In this way, unhybridised (labelled) probes can be separated from (labelled) probe/target complexes (illustrated in Figure 1), and non-target double stranded DNA (where applicable).

Accordingly, the methods according to this aspect of the invention comprise the step of applying a well-defined current for a given period of time to the reaction chamber, such that the separation of probe/target complex and unhybridised probes is achieved.

The present invention envisages embodiments wherein the probe and/or target molecules are modified to obtain (or further increase) a different or opposite mobility of unbound labelled probe and probe-target complex.

More particularly, specific embodiments of the methods of the present invention envisage the modification of the labelled nucleic acid probe with a moiety which carries a number of positive charges, such that the net charge of the modified labelled probe is positive. Upon application of an electric current to a solution comprising both modified unbound labelled probe and probe/target complex, the modified unbound labelled probe moves to the negative electrode, as a result of the positive charges, while all other nucleic acids, including the probe/target complex, which still has an excess negative charge, move towards the positive electrode.

According to another embodiment of the methods of the present invention, physical separation of the unbound probe and the probe/target complex by making use of a manipulatable label.

In particular embodiments, the methods of the present invention envisage attaching the manipulatable label to the target. For instance, where the target is DNA, a manipulatable label, such as a magnetic particle can be incorporated into the amplified target by PCR, making use of a PCR primer to which a manipulatable label has been attached as described above.

This is ensured, for instance, by using biotinylated primers. Magnetic beads functionalised with avidin are then used to bind to the biotinylated nucleic acid.

Alternatively, manipulatable labels can be attached to target DNA without amplification of the target nucleic acid via the binding of e.g. magnetic particles to the 3' or 5' of nucleic acids, analogous to the labelling of mRNA in micro array labelling techniques (e.g. Richter et al. (2002) Biotechniques 33, 620-628 and Xiang et al. (2002) Nat. Biotechnol. 20, 738-742).

In alternative embodiments, the target is provided with a manipulatable probe by attaching the manipulatable probe to the target using a capture molecule. These alternative embodiments are of particular interest where the nature of the molecules within the sample is homogenous and a suitable capture molecule can be used to bind with the target in a sample.

In the above embodiments, the methods of the invention comprise the step of incorporating a manipulatable label into the target DNA. Accordingly, the step of manipulating the probe/target to the detection zone of the reaction chamber comprises applying a force or field which ensures the manipulation of the manipulatable label on the target DNA. More particularly, where the manipulatable label is a magnetic particle and the detection is based on detection of the probe/target complex, the step of manipulating the probe/target to the detection zone of the reaction chamber comprises the application of a magnetic field within the reaction chamber such that the magnetic particles are drawn to the detection zone of the reaction chamber.

The methods of the present invention further comprise the detection of the target with a chromophoric label. This chromophoric label is bound to the target by way of a probe or primer comprising the chromophoric label.

According to particular embodiments, the methods of the present invention comprise hybridizing the target, in solution, to a target-specific probe comprising a chromophoric label. Upon hybridization of the target with the labelled probe, a probe/target complex is formed. Typically, the detection of the labelled probe/target complex is used as a measure of the presence and amount of target in the sample. Alternatively, the amount of unbound labelled probe remaining after hybridization is detected and taken as a measure of the presence and the amount of target in a sample. In both embodiments of the methods of the present invention, the labelled probe/target complex and/or unbound labelled probe are manipulated to ensure physical separation prior to detection, using the methods described above.

According to a further particular embodiment of the methods of the present invention, the target is contacted with a manipulation probe comprising a manipulatable label and a detection probe comprising a chromophoric label (the latter forming the probe-target complex). The manipulation probe and the detection probe are designed such that they both specifically bind to a different region of the target nucleic acid.

According to particular embodiments, the chromophoric label is incorporated into the target DNA by PCR. This can be ensured by amplifying the target nucleic acid or a fragment thereof with a primer comprising a chromophoric label.

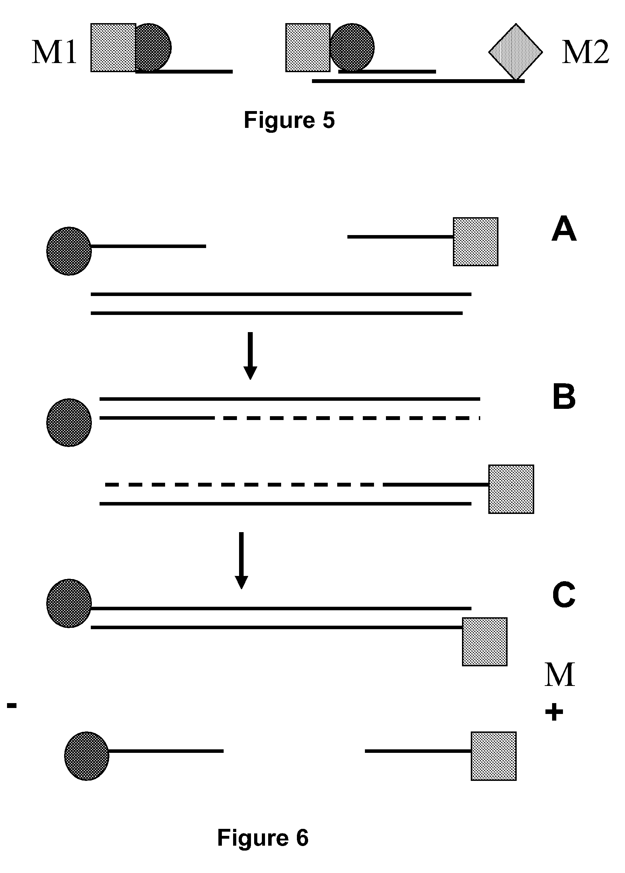

In a particular embodiment of the methods of the present invention, the chromophoric label and manipulatable label are each provided on a different oligonucleotide primer and are incorporated into the target nucleic acid in a PCR reaction (Illustrated in Figure 6). In one embodiment, the first of a set of PCR primers is functionalised with a manipulatable label (e.g. a magnetic particle) while the second of the set of PCR primers is provided with a chromophoric label. Using these primers, amplified DNA will comprise both the manipulatable label and the chromophoric label. Upon application of the appropriate external field (e.g. magnetic field) the probe/target complex (i.e. amplified DNA) and the unbound manipulation probe, both comprising a manipulatable label are moved in the direction of the magnetic field. In particular embodiments, the methods of the present invention comprise applying a magnetic field to the reaction chamber comprising a detection zone and a non-detection zone such that magnetic particles are moved from one zone to another.

The methods of the present methods comprise the step of manipulating unbound labelled detection probe and/or probe/target complex such that either only the unbound detection probe or only the labelled probe/target complex is present in the detection zone of the reaction chamber. Accordingly, manipulation involves application of an external force or field such that the unlabelled detection probe and/or probe/target complex are moved towards or away from the detection zone but remain in a zone fluidly connected to the detection zone.

Accordingly, as a result of the manipulation step of the methods of the invention the detection zone of the reaction chamber comprises either unlabelled detection probe or labelled probe/target complex. Depending on the method, other molecules may also concentrate in the detection zone of the reaction chamber, for example other sample nucleic acids. This is the case, e.g., when the methods of the invention encompass manipulating the labelled unbound probe away from the detection zone (using an electric current) and detection is performed on a rough sample. Similarly, in those embodiments of the methods of the present invention which comprise the non-specific labelling of target nucleic acids with a manipulatable label and the manipulation of the probe/target complex towards the detection zone based on the manipulatable label (e.g. magnetic bead). However, these nucleic acid molecules originating from the sample do not carry a chromophoric label and thus do not disturb the detection.

Different embodiments of the physical separation methods of unbound labelled probe and probe/target complex described above can be combined or inversed to further improve the physical separation and thus accuracy of detection. Thus, the present invention also envisages embodiments wherein only the unbound probe is actively manipulated or wherein both unbound probe and probe/target complex are manipulated in different directions. This latter embodiment is particularly suitable when the chromophoric labelled probe needs to be present in large excess and/or the concentration of probe-target complex is low. The manipulation of the probe can be performed using intrinsic properties of the probe, or can be performed by attaching a manipulatable label to the probe.

In what follows, a number of straightforward embodiments of the methods of the present invention are summarized and illustrated.
(1) Target DNA is hybridized with a labelled probe and unbound labelled probe and probe/target complex are separated by application of an electrical field (Figure 1).
(2) Target DNA is labelled with a manipulatable label, i.e. a magnetic particle, and is then hybridized with a detection probe. Unbound labelled probe and probe/target complex are separated by application of a magnetic field (Illustrated in Figure 2). In this configuration,
(3) Target DNA is hybridized with one manipulation probe (e.g. with a magnetic label) and another detection probe with a chromophoric label. Upon application of a magnetic field, the unbound chromophoric labelled probe remains homogeneously present in the reaction chamber, while the probe-target complex is concentrated in the detection zone of the reaction chamber for optical detection.
(4) Target nucleic acids are bound to magnetic particles and probe-target complex is separated from the unbound labelled probe by applying both an electrical current and a magnetic field. The unbound labelled probe moves towards the positive electrode while the probe/target complex is manipulated to the desired location by the magnetic field (Illustrated in Figure 3). In these embodiments the magnetic field applied is sufficiently large to counter the effect of the electric current on the probe-target complex.
(5) Both chromophoric detection probe and target nucleic acid are modified with a manipulatable label. The chromophoric detection probe is provided with a first manipulatable label (e.g. glass bead) and the target nucleic acid is modified by attaching a second manipulatable label, which is different from the first manipulatable label (e.g. a magnetic label). Application of optical tweezers and a magnetic field ensures the separation of unbound labelled probe and probe-target complex (Illustrated in Figure 5).
(6) Only the chromophoric labelled probe comprises a manipulatable label. The unbound labelled probe is manipulated via the manipulatable label. To ensure separation, the probe/target complex is simultaneously manipulated to a different direction using the intrinsic properties of the target nucleic acid. This can be done by applying an electric field which manipulates the probe/target complex to the desired location (Illustrated in Figure 4).

The methods of the invention comprise the contacting of target, labelled detection probes or primers and optionally manipulatable probes or primers in a reaction vessel, followed by detection in a detection zone of the detection vessel. Suitable devices for carrying out the methods of the present invention are detailed below. In particular embodiments, the methods of the present invention are carried out in a reaction chamber comprising a detection zone and at least a second zone, which are in fluid contact with each other (i.e. fluids move freely from one zone to another, but which are physically separated by a membrane and/or a connecting zone. This allows the contacting of the target with the probe(s) in the non-detection zone and ensures that, when e.g. the labelled probe/target complex is manipulated towards the detection zone, the unlabelled probe remains (essentially) in the non-detection zone. Alternatively it is envisaged that the methods of the invention are carried out in a reaction chamber whereby the detection zone is not physically separated from the rest of the reaction chamber. In these embodiments, labelled probe and probe/target complex will move freely around the whole area of the reaction chamber and, upon manipulation of the probe/target complex so as to enrich the probe/target complex in the detection zone, unbound labelled probe will still be present in the detection zone. The unbound labelled probe then contributes to the optical signal with a background signal. This is optionally solved by providing a second region for optical detection at a place remote from the detection zone (used for detecting the signal of the probe/target complex), to measure the background signal of the unbound labelled probe. The signal of the unbound labelled probe is subtracted from the signal of the probe/target complex to obtain the real signal of the probe/target complex. Alternatively, the unbound probe can be manipulated based away from the detection zone by application e.g. of an electric field as described hereinabove.

The methods of the present invention enable the performance of multiplexing assays. Indeed a sample comprising different target nucleic acids can be analysed within the same assay, when different detection probes for detecting the different target nucleic acids are used, each carrying a different chromophoric label, i.e. which adsorbs at a different wavelength.

The methods of the present invention are suited for all types of nucleic acid detection assays, such as mutation detection or SNP detection. For example, SNP detection can be ensured by using a probe hybridising with wild type DNA (probe A) which is unlabelled or labelled with a chromophoric group and a probe specifically hybridising with the mutant DNA (probe B), which is labelled with a chromophoric group, which is different from the one on probe A. The double stranded probe/target DNA is separated from the unbound probes by use of a probe with a manipulatable label which binds in the proximity of the DNA where the mutation is present, or alternatively by incorporating a manipulatable label in the target by PCR. The presence or nature of the chromophoric label detected in the probe/target complex is indicative of the presence of a SNP. Such methods find their application in e.g. DNA fingerprinting for forensic research.

In particular embodiments, the methods of the present invention are used in real time quantitative PCR assays. Herein the sample is analysed after each cycle by temporarily moving the PCR product and/or free PCR primer to the detection zone in the reaction chamber. After the detection, primers and nucleic acids are brought back into contact with each other, whereafter a new round of amplification can take place.

Using the methods of the present invention, the analysis of different target amplifications can take place within the same PCR reaction chamber, as for each target different primers are used carrying a different chromophoric label. This is of interest, e.g. to discriminate between closely related microorganisms within a biological sample, for example to discriminate between pathogenic and non-pathogenic strains of a same bacterium, whereby strain specific primers are labelled with different chromophoric labels.

The above section provides particular embodiments of the invention, as envisaged in the detection of nucleic acids. The methods of the present invention are however also applicable to the detection of other biological target molecules such as proteins or peptides. Generally, the term 'oligopeptide or peptide' is used to designate peptides with a length up to 50 amino acids. 'Polypeptide' refers to peptides with a length above 50 amino acids. The term 'protein' generally refers to a peptide with a biological function. Most proteins have a length above 50 amino acids. Peptides are charged molecules because of the presence of an NH₂ and a COOH group at respectively the amino- and carboxyterminus and because of the presence of the charged side chains of Asp, Glu, Lys, Arg and His. Accordingly, proteins can be manipulated by electric currents in the assays of the present invention, similar to what is described above for nucleic acids. The behaviour of a protein in an electrical field is dependent on the relative amounts of the charged amino acids in the protein and may vary considerable from protein to protein. The mobility of a protein in an electric field can further be influenced by changes in pH and the direction of mobility can be even reversed by shifting the pH to a value above or below its isoelectric point. Even when a protein has an overall net charge, proteins can be manipulated based on their dielectric properties.

Suitable probes for the detection of proteins according to the methods of the present invention vary in nature and include antibodies or antigen binding portions thereof, receptors or ligand binding portions thereof, protein ligands of receptors or the receptor binding portions thereof. Other probes with specific protein binding properties include ligands, inhibitors, modified enzyme substrates and the like. Such probes can be organic small molecules or can be of (modified) peptide nature.

Similar to what is described for nucleic acids above, the manipulation of probe and probe/target protein complex is in particular embodiments of the methods of the present invention performed using the intrinsic properties of the probe and/or probe/target protein. Additionally or alternatively, manipulation is ensured by the attachment of a manipulatable label to probe and/or to the target. Different functional groups on a protein (typically NH₂, COOH, SH) are suitable for the attachment of a chromophoric or manipulatable label thereto.

In a particular embodiment the pH of a sample is selected such that the probe/target complex moves to the detection zone and/or the unbound probe moves to a non-detection zone within the reaction chamber upon application of an electric field. Additionally or alternatively the charge of polypeptide probes is modified by the incorporation of e.g. additional charged amino acids in the probe.

Contrary to nucleic acid detection, where target nucleic acids have similar physicochemical properties, protein target are more divers in terms of charge. On the other hand, while nucleic acids are generally detected with oligonucleotides, a large variety of peptide and non-peptide probes are available for the formation of the probe/target complex using the methods of the present invention. For each pair of probe and target molecule, modifications of probe and/or target can be envisaged to enable the separation of unbound probe and probe/target complex by applying the appropriate external field as described herein.

A further aspect of the present invention relates to devices for performing the methods of the present invention. Such devices (1) comprise an optionally removable reaction chamber (5), suitable for containing a fluid sample, comprising a separate detection zone (52) and a non-detection (or control detection) zone (51), means for manipulation (2) spanning the detection zone (52) and the non-detection zone (51), and means for optical detection in said detection zone (3).

The means for manipulation include, but are not limited to, for example an electric current generator, a magnetic field generator or optical tweezers. These are positioned such that the field is generated between the detection zone and the non-detection zone in the reaction chamber, such that, upon application of the manipulation means, manipulatable molecules are moved between these two zones.

The reaction chamber (5) is optionally removable placed within a reaction chamber holder (4) and is suitable for containing a reaction fluid. Typically it comprises at least one region which is light-transparent (6) to enable the optical detection of a chromophoric label in the detection zone of the reaction chamber. Optical detection can be done by any type of spectrophotometer used for the detection of chromophoric labels. Optionally, the devices of the present invention can further comprise (instead of or in addition to the non-detection zone) a second detection zone, remote from the first detection zone, which can be used for detecting a background signal. This is of particular interest where the detection of the signal is performed in a reaction chamber wherein the detection zone is not physically separated.

Fluorescent or Surface-enhanced (resonance) Raman (SE(R)RS) emission signal may be detected by a Fluorescence and/or Raman detector. A variety of detection units of potential use in Raman spectroscopy are known in the art and any known Raman detection unit may be used. An example of a Raman detection unit is disclosed e.g. in U.S. Pat. No. US 6002471. Other types of detectors may be used, such as a charge coupled device (CCD), with a red-enhanced intensified charge-coupled device (RE-ICCD), a silicon photodiode, or photomultiplier tubes arranged either singly or in series for cascade amplification of the signal. Photon counting electronics can be used for sensitive detection. The choice of detector will largely depend on the sensitivity of detection required to carry out a particular assay. Several devices are suitable for collecting SE(R)RS signals, including wavelength selective mirrors, holographic optical elements for scattered light detection and fibre-optic waveguides.

The devices of the present invention optionally further comprise additional parts such as heating/cooling elements to denature/renature DNA or to perform PCR reactions.

Suitable devices for performing the methods of the present invention are for example conventional PCR thermocyclers wherein dye-quencher PCR amplification takes place and which are modified to contain reaction chambers comprising a separate detection zone and for the purpose of the methods of the present invention and a manipulation means e.g. a magnetic field generator around the reaction chamber.

The reaction chamber envisaged in the devices of the methods of the present invention can vary in geometry or design, as long as a detection zone and a non-detection zone is present. Thus, at least part of the reaction chamber wall is transparent for optical detection of the presence of a (bound or unbound) chromophoric label in the detection zone of the reaction chamber. In one embodiment the reaction chamber is a reaction vial with no further compartments.

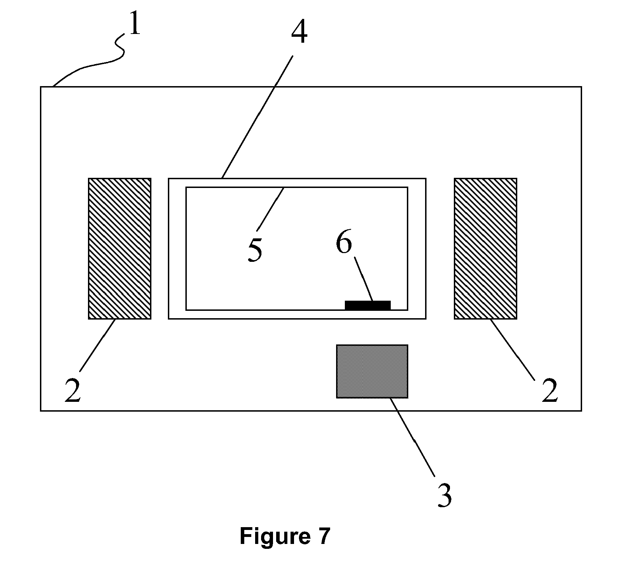

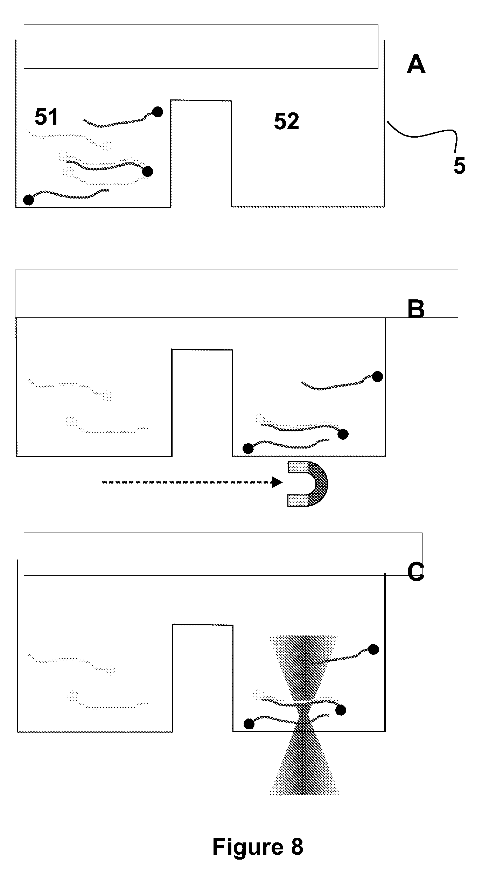

According to some embodiments the reaction chamber (5) is to some extent divided into compartments as indicated in Figure 8. Herein a minimum of diffusion takes place from the non-detection zone or hybridization chamber (51) (typically, but not necessarily comprising the unbound labelled probe) to the detection zone or detection chamber (52) (typically comprising the probe/target complex) such that there is no or a limited amount of background caused. In particular embodiments it is envisaged that the device is configured to minimize influence of remixing of the sample by diffusion. In order to limit remixing of the sample due to diffusion, the distance between the detection zone and the non-detection zone is chosen to be larger than the average diffusion distance of the unbound probe molecule during the time needed for optical detection. This distance is optimised based on the size of the probe molecule and the integration time of the optical measurement for detection. Typically, the distance will be between 10 µm and 1 mm with an integration time of about 1-10 s for the detection. Alternatively, a fluid flow may be generated to flush unbound molecules away from the detection zone. Reaction chambers such as depicted in Figure 7 or 8 can further comprise means to temporarily interrupt the fluid connection between the reaction compartment.

Other arrangements of the systems and methods embodying the invention will be obvious for those skilled in the art.

It is to be understood that although preferred embodiments, specific constructions and configurations, as well as materials, have been discussed herein for devices according to the present invention, various changes or modifications in form and detail may be made without departing from the scope and spirit of this invention.

### EXAMPLES

### Example 1: Detection of HIV

Blood of a human patient is diluted 1/1000 in TE buffer, boiled for 5 minutes and centrifuged. The presence of HIV virus is determined by PCR using 5 µl of the sample supernatant. Concentrations of buffer, dNTPs, magnesium and Taq Polymerase are as indicated by the manufacturer. Primers used for the amplication are a Cy3 labelled forward primer (5'- ggagctagaacgattcgcagtta-3') and a 5' NH₂ modified reverse primer (5'-ggttgtagctgtcccagtatttgtc-3') for amplifying a portion of the HIV *gag* gene. The NH₂ modification is used to covalently couple the reverse primer to a carboxyl magnetic bead (Dynabead, Dynal). Fluorescence is monitored in two different detection zones within the reaction chamber during the PCR reaction: near the end of each elongation step an external magnetic field is applied which concentrates the double stranded DNA and the magnetic labelled primer in the first detection zone. In the second detection zone, the background signal of the labelled primer is measured. The concentration of amplified PCR product is determined by subtracting the fluorescence signal of the second detection zone (background signal) from the first detection zone. After each measurement, random changing of the magnetic field is applied to ensure redistribution of the magnetically labelled primer and the amplified DNA in the reaction chamber.

## Claims

1. A method for detecting the presence of a target in a sample, comprising the steps of,
a) contacting said sample comprising said target with a optically labelled detection probe in a sample container comprising a detection zone,
b) allowing said probe to bind with said target so as to form a probe/target complex,
c1) manipulating the probe/target complex or unbound probe to the detection zone, and/or
c2) manipulating unbound probe or probe/target complex, respectively away from the detection zone, and
d) optically detecting the probe/target complex or the unbound probe as a measure of the amount of target in the sample,
wherein the probe/target complex and the unbound probe remain in solution within the sample container.

2. The method according to claim 1, wherein said manipulation of steps c1 and/or c2 is performed by applying an electric field on the reaction chamber.

3. The method according to claim 1 or 2, wherein, prior to step (a) the target is provided with a manipulatable label.

4. The method according to any of claims 1 to 3, which further comprises contacting the target with a manipulation probe comprising a manipulatable label, whereby the detection probe and the manipulation probe each bind specifically to a different region of the target.

5. The method according to claim 3, wherein the manipulatable label is a magnetic particle.

6. The method according to claim 3, wherein the manipulatable label is a glass or polystyrene bead.

7. The method according to any of claims 1 to 6, wherein the target and the optically labelled probe are nucleic acids.

8. The method according to claim 7, wherein the target nucleic acid is amplified by PCR prior to step (a).

9. The method according to claim 8, wherein in said PCR, a PCR primer pair is used of which one primer comprises a chromophoric label and the other primer comprises a manipulatable label and wherein the probe/target complex is comprised within the amplified target.

10. The method according to any of claims 1 to 6, wherein the target is a peptide or protein.

11. The method according to claim 10, wherein the optically labelled probe is an optically labelled antibody or antigen binding part thereof.

12. A primer pair for performing a PCR reaction wherein one primer of the pair comprises a chromophoric label and wherein the second primer comprises a manipulatable label.

13. The primer pair according to claim 12 wherein the manipulatable label is a magnetic particle.

14. A device (1) for qualitatively and/or quantitatively detecting a target in a sample, said device comprising:
- an optionally removable reaction chamber (5), suitable for containing a fluid sample, comprising a separate detection zone (52) and a non-detection zone or control detection zone (51)
- manipulation means (2) spanning the detection zone (52) and the non-detection or control detection zone (51) of the reaction chamber, and
- means for optical detection (3) in said detection zone (52).

15. The device according to claim 14, wherein said manipulation means (2) is a means for applying an electric or magnetic field between the detection zone (52) and the non-detection zone (51).

16. The device according to claim 14, wherein the non-detection zone and the detection zone are comprised within separate chambers, which are fluidly connected.

17. The device according to claim 14, further including an element for cooling and heating the reaction chamber between 4 and 100 °C.

18. The device according to claim 14, wherein said manipulation means (2) is a means for applying a light beam to move the manipulatable label between the detection zone (52) and the non-detection zone (51).
